# EUROPÄISCHE PATENTSCHRIFT

(11) **EP 2 663 274 B1**
(45) Veröffentlichungstag und Bekanntmachung des Hinweises auf die Patenterteilung: **04.01.2017**
(21) Anmeldenummer: 12700121.2
(22) Anmeldetag: 10.01.2012
(51) Int. Cl.: A61J 1/03, A61J 1/10

(54) **VERPACKUNG, ENTHALTEND EIN MEDIZINISCHES PRODUKT ZUR BEHANDLUNG VON MENSCHLICHEN ODER TIERISCHEN KNORPELSCHÄDEN**
PACKAGING CONTAINING A MEDICAL PRODUCT FOR TREATING CARTILAGE DAMAGE IN HUMANS OR ANIMALS
EMBALLAGE CONTENANT UN PRODUIT MÉDICINAL POUR LE TRAITEMENT DES LÉSIONS DU CARTILAGE HUMAIN OU ANIMAL

(30) Priorität: 11.01.2011 DE 102011002536
(43) Veröffentlichungstag der Anmeldung: 20.11.2013
(73) Patentinhaber: Aesculap AG, 78532 Tuttlingen (DE)
(72) Erfinder: KÖNIG, Silke, 78628 Rottweil (DE); BLENDER, Bernd, 78532 Tuttlingen (DE)
(74) Vertreter: Patentanwälte Ruff, Wilhelm, Beier, Dauster & Partner mbB
(86) Internationale Anmeldenummer: PCT/EP2012/050314
(87) Internationale Veröffentlichungsnummer: WO 2012/095426

(56) Entgegenhaltungen:
- EP-A1- 1 049 415
- AU-B2- 515 643
- US-A1- 2004 172 007
- US-A1- 2006 276 769
- US-A1- 2008 077 251
- US-A1- 2010 155 282

## Beschreibung

Die Erfindung betrifft eine Verpackung, welche ein medizinisches Produkt enthält, wobei das medizinische Produkt zur regenerativen Behandlung von menschlichen oder tierischen Knorpelschäden, vorzugsweise Meniskusschäden, vorgesehen ist.

Der Meniskus stellt ein kompliziertes ring- bzw. scheibenförmiges Knorpelgewebe dar, welches im Gegensatz zu einem Diskus die Gelenkhöhle nur unvollständig teilt. Der Innenmeniskus (meniskus medialis) und der Außenmeniskus (meniskus lateralis) des Kniegelenks sind als paarige C- bzw. halbmondförmige Faserknorpelscheiben ausgebildet. Ihre Aufgabe besteht in der Stabilisierung des Kniegelenks. Hierzu gleichen sie Inkonkruenzen zwischen den artikulierenden Gelenkknochen, dem Oberschenkelknochen (Femur) und dem Schienbein (Tibia), durch Aufnahme von Druck- und Scherkräften aus.

Da der Innenmeniskus im Gegensatz zum Außenmeniskus fest mit der Gelenkkapsel und dem Innenband verwachsen ist, ist er grundsätzlich verletzungsanfälliger als der Außenmeniskus.

Meniskusverletzungen, beispielsweise nach Stürzen und/oder Unfällen, manifestieren sich in der Regel in Form von Rissen, welche an der konvexen Außenseite des Meniskus, an der konkaven Innenseite des Meniskus oder dazwischen lokalisiert sein können.

Während außen liegende Risse in der Regel genäht werden können, wird an der Innenseite des Meniskus ein gerissenes Gewebe ausgeräumt bzw. weggeschnitten. Problematisch hierbei ist, dass der verbleibende Meniskus in der Regel nicht mehr in der Lage ist, das ausgeräumte bzw. abgeschnittene Gewebe zu ersetzen. Hinzu kommt, dass eine operative Teilentfernung des Meniskus eine fortschreitende Degeneration des verbleibenden Meniskus zur Folge haben kann, wodurch das Risiko, an Arthrose zu erkranken, für die betroffenen Patienten steigt.

Deswegen werden in den letzten Jahren in zunehmendem Maße regenerative Methoden zur Behandlung von Meniskusschäden eingesetzt. Diese Methoden zielen darauf ab, die originäre Meniskusfunktionalität wiederherzustellen, indem die Neubildung von Knorpelgewebe stimuliert wird. Dadurch kann eine Arthrose grundsätzlich vermieden oder deren klinische Manifestation wenigstens verzögert werden.

Bei Patienten mit weit ausgedehnten Meniskusschäden besteht die einzige Behandlungsmöglichkeit jedoch häufig darin, den geschädigten Meniskus vollständig zu entfernen und entweder durch einen Spendermeniskus oder ein sogenanntes regeneratives Implantat, d.h. ein Implantat, welches den Aufbau bzw. die Synthese von neuem Gewebe, insbesondere Knorpelgewebe, induziert oder fördert, zu ersetzen. Aufgrund ihrer allogenen Natur bestehen im Falle von Spendermenisken jedoch gewisse Bedenken hinsichtlich ihrer Immunogenität und ihrem Langzeitverhalten im Körper des Empfängers.

Um den Neuaufbau von körpereigenem Knorpelgewebe zu induzieren bzw. zu fördern, werden für die regenerative Behandlung vorgesehene Implantatstrukturen in der Regel mit autologen Knorpelzellen (Chondrozyten) beimpft und anschließend als regenerative Implantate wieder in die Patienten implantiert. Eine grundsätzliche Schwierigkeit hierbei besteht darin, dass in der Regel lediglich eine limitierte Menge an autologen Chondrozyten für die Beimpfung der Implantatstrukturen zur Verfügung steht. Hinzu kommt, dass sich die für eine regenerative Knorpelbehandlung vorgesehenen Implantatstrukturen sehr häufig durch eine heterogene Verbundstruktur auszeichnen. Insgesamt besteht daher ein gewisses Risiko, dass auch bei längerer Inkubation bzw. Kultivierung mit Knorpelzellen lediglich eine unvollständige Besiedelung der Implantatstrukturen erzielt wird, was den Neuaufbau von körpereigenem Knorpelgewebe beeinträchtigen kann.

Aus der US 2006/0276769 A1 ist ein Container bzw. Beutel zur Aufbewahrung von medizinischen Flüssigkeiten bekannt.

Die EP 1 049 415 A1 betrifft eine Doppelverpackung mit einem inneren Container und einem äußeren Container, wobei der innere Container ein zellhaltiges Material enthalten kann.

Gegenstand der AU 515 643 B2 ist ein Behältnis für die Aufbewahrung und Verabreichung einer parenteralen Lösung.

Die US 2004/0172007 A1 bezieht sich auf ein Behältnis für die Bestrahlung von Blutprodukten.

Die US 2008/0077251 A1 offenbart eine Doppelverpackung mit einem inneren Verpackungsmittel und einem äußeren Verpackungsmittel, wobei das innere Verpackungsmittel ein Knorpelimplantat enthält.

Aus der US 2010/0155282 A1 sind Verpackungssysteme für medizinische Implantate bekannt, wobei die Implantate in den Verpackungssystemen unter Unterdruck bevorratet sein können.

Die vorliegende Erfindung stellt sich daher die Aufgabe, eine Verpackung bereitzustellen, welche ein medizinisches Produkt verfügbar macht, welches sich zur regenerativen Behandlung von Knorpelschäden wie beispielsweise Meniskusschäden eignet, und aus dem Stand der Technik bekannte Nachteile umgeht. Dabei soll die Verpackung eine möglichst einfache, effiziente und insbesondere homogene Besiedelung eines in der Verpackung enthaltenen medizinischen Produktes mit Zellen und/oder Wirkstoffen ermöglichen.

Diese Aufgabe wird erfindungsgemäß gelöst durch eine Verpackung, insbesondere nach Art eines Beutels, bevorzugt nach Art eines Infusionsbeutels, welche ein medizinisches Produkt, insbesondere zur regenerativen Behandlung von menschlichen und/oder tierischen Knorpelgewebsschäden, höchst bevorzugt Meniskusschäden, enthält. Die Verpackung weist zudem eine Komponente zur Befüllung der Verpackung mit einer Flüssigkeit auf.

Die erfindungsgemäße Verpackung, insbesondere die erfindungsgemäß vorgesehene Komponente zur Befüllung der Verpackung, im Folgenden auch als Befüllungskomponente bezeichnet, erlaubt mit besonderem Vorteil eine Inkubation, insbesondere Benetzung und/oder Durchtränkung, eines noch im verpackten Zustand befindlichen medizinischen Produktes mit einer Flüssigkeit. Mit anderen Worten kann die Verpackung mit besonderem Vorteil als eine Art Inkubationsbehältnis für das Produkt und eine Flüssigkeit, mit welcher das Produkt inkubiert werden soll, verwendet werden.

Bei der Flüssigkeit handelt es sich bevorzugt um eine medizinisch wirksame, insbesondere therapeutisch wirksame, Flüssigkeit. Auf diese Weise ist es möglich, das medizinische Produkt vor seiner Entnahme aus der Verpackung indikationsspezifisch mit bestimmten medizinischen bzw. therapeutischen Eigenschaften zu versehen.

Die Flüssigkeit ist in einer weitergehenden Ausführungsform eine wirkstoff- und/oder zellhaltige, vorzugsweise eine zellhaltige, Flüssigkeit. Enthält die Flüssigkeit beispielsweise Zellen wie autologe Körperzellen, so wirkt die erfindungsgemäße Verpackung mit besonderem Vorteil als eine Art Kultivierungsbehältnis für das in der Verpackung enthaltene medizinische Produkt sowie (nach Befüllung der Verpackung mit der zellhaltigen Flüssigkeit) für die in der Verpackung enthaltenen Zellen. Zur Kultivierung von Zellen in der Verpackung kann es zum Beispiel vorgesehen sein, die Verpackung zusätzlich mit geeigneten Nährstofflösungen zu befüllen. Auf geeignete Wirkstoffe und/oder Zellen wird im Folgenden noch näher eingegangen.

Die Verpackung ist in einer bevorzugten Ausführungsform als Beutel ausgebildet. Besonders bevorzugt handelt es sich bei der Verpackung um einen Kunststoffbeutel.

Die Verpackung ist in einer weiteren bevorzugten Ausführungsform aus einer Folie, insbesondere einer Flach- oder Schlauchfolie, hergestellt. Die Folie kann als einlagige oder mehrlagige, insbesondere zwei- oder dreilagige, Folie ausgebildet sein.

In einer besonders bevorzugten Ausführungsform ist die Verpackung aus einer Kunststofffolie, insbesondere einer zweilagigen Kunststofffolie, hergestellt.

Bei der Verpackung handelt es sich in einer weiteren Ausführungsform um einen Kunststofffolienbeutel, insbesondere um einen zweilagigen Kunststofffolienbeutel.

Die Verpackung bzw. die in den vorherigen Ausführungsformen genannte Kunststofffolie ist in einer weitergehenden Ausführungsform aus einem Kunststoff hergestellt, welcher ausgewählt ist aus der Gruppe bestehend aus Polyolefine, Polyamide, Copolymere davon und Mischungen, insbesondere Blends, davon.

Besonders bevorzugt ist die Verpackung bzw. die Kunststofffolie aus einem Kunststoff hergestellt, welcher ausgewählt ist aus der Gruppe bestehend aus Polyethylen, Polyethylen hoher Dichte (HDPE), Polypropylen, Polypropylen hoher Dichte (HDPP), Copolymere davon, insbesondere Ethylen-Vinylalkohol, und Mischungen, insbesondere Blends, davon.

Bei der Verpackung handelt es sich zweckmäßigerweise um eine verschlossene, insbesondere versiegelte, bevorzugt verschweißte, Verpackung. Erfindungsgemäß ist es dabei insbesondere vorteilhaft, wenn die Verpackung keimdicht, gasdicht und insbesondere feuchtigkeitsdicht verschlossen ist.

In einer besonders bevorzugten Ausführungsform liegt die Verpackung verschweißt vor. Zur Verschweißung der Verpackung kommen bevorzugt Kunststoff-Schweißverfahren, wie beispielsweise Heizelement-Schweißen, Induktions-Schweißen, Laserstrahl-Schweißen, IR-Licht-Schweißen, Warmgas-Schweißen, Extrusions-Schweißen, HochFrequenz-Schweißen, Ultraschall-Schweißen, Vibrations-Schweißen oder dergleichen in Betracht. Derartige Schweißverfahren sind dem Fachmann an sich bekannt, so dass auf diese Verfahren im Einzelnen nicht näher eingegangen wird.

Erfindungsgemäß ist es insbesondere vorgesehen, dass die Verpackung entlang eines Falzes, insbesondere entlang eines randseitig verlaufenden Falzes, verschweißt ist.

Die Befüllungskomponente weist in einer weiteren Ausführungsform einen Kunststoffkörper auf. Der Kunststoffkörper ist vorzugsweise zwischen wenigstens zwei, bevorzugt zwei, Lagen, insbesondere zwischen wenigstens zwei, bevorzugt zwei, Kunststofffolienlagen, der Verpackung eingeschweißt.

Bei der erfindungsgemäß vorgesehenen Komponente zur Befüllung der Verpackung handelt es sich in einer weitergehenden Ausführungsform um einen Port. Insbesondere kann der Port einen Kunststoffkörper aufweisen, der bevorzugt zwischen zwei Lagen, insbesondere zwischen zwei Kunststofffolienlagen, der Verpackung, vorzugsweise unter Ausbildung eines Portschweißbereiches, eingeschweißt ist.

Der Kunststoffkörper ist typischerweise aus einem Polyolefin wie beispielsweise Polypropylen hergestellt.

Besonders bevorzugt weist die Verpackung einen Portschweißbereich und einen Falzschweißbereich, insbesondere einen randseitig verlaufenden Falzschweißbereich, auf. Bevorzugt geht der Falzschweißbereich im Bereich eines zwischen wenigstens zwei, insbesondere zwei, Lagen der Verpackung liegenden Ports, in der Regel fließend, in einen Portschweißbereich über.

In einer alternativen Ausführungsform ist die Verpackung als Blisterverpackung (Sichtverpackung), insbesondere als Tiefziehblisterverpackung (Tiefziehsichtverpackung), ausgebildet.

Die Blisterverpackung weist in der Regel ein Kunststoffformteil, insbesondere ein Kunststofftiefziehformteil, vorzugsweise mit einer Gehäuseform, auf.

Das Kunststoffformteil kann aus einem Kunststoffmaterial wie beispielsweise Polyethylenterephthalat (PET) und/oder Polycarbonat gebildet sein.

Das Kunststoffformteil kann weiterhin insbesondere folienartig bzw. als Folie ausgebildet sein.

In einer weiteren Ausführungsform weist die Blisterverpackung eine gasdurchlässige und insbesondere keimdichte Abdeckschicht auf. Die Abdeckschicht wirkt mit besonderem Vorteil als eine Art Sterilisationsfenster, in dem sie den Durchtritt von Sterilisationsgasen wie beispielsweise Ethylenoxid-Gas in das Verpackungsinnere ermöglicht. Nach Beendigung des Sterilisationsvorganges kann das Sterilisationsgas aus der Verpackung, beispielsweise durch Anlegen eines Vakuums oder Unterdrucks, aufgrund der Gasdurchlässigkeit der Abdeckschicht wieder entfernt werden.

In einer besonders bevorzugten Ausführungsform weist die Verpackung eine gasdurchlässige und insbesondere keimdichte Abdeckschicht mit einer vliesartigen, insbesondere papiervliesartigen, Struktur auf.

Die Abdeckschicht kann insbesondere aus einem Polyolefin wie beispielsweise Polyethylen, insbesondere Polyethylen hoher Dichte (HDPE), Polypropylen, insbesondere Polypropylen hoher Dichte (HDPP), Copolymere davon und Mischungen, insbesondere Blends, davon, gebildet sein.

Erfindungsgemäß kann es bevorzugt sein, wenn die Abdeckschicht folienartig bzw. als Folie (Abdeckfolie) ausgebildet ist.

Gemäß einer besonders bevorzugten Ausführungsform ist die Abdeckschicht ein vliesartiges, insbesondere papiervliesartiges, Faserfunktionstextil, vorzugsweise aus thermisch verschweißten Fasern. Eine erfindungsgemäß geeignete Abdeckschicht kann beispielsweise aus einem kommerziell unter der Bezeichnung Tyvek^{®} erhältlichen Material hergestellt sein. Hierbei handelt es sich um ein papiervliesartiges Faserfunktionstextil aus thermisch verschweißten Fasern aus Polyethylen hoher Dichte (HDPE).

In einer weiteren Ausführungsform weist die Blisterverpackung eine gasdichte und insbesondere feuchtigkeitsdichte Deckschicht auf. Auf diese Weise ist eine trockene Lagerung des medizinischen Produktes in der Blisterverpackung möglich.

Die Deckschicht besteht in einer weitergehenden Ausführungsform aus Aluminium oder einem Verbundmaterial mit Aluminium. Eine Deckschicht aus oder mit Aluminiumlegierungen ist erfindungsgemäß ebenfalls denkbar.

Bei der Deckschicht kann es sich in einer weiteren Ausführungsform um ein Verbundmaterial mit einem schichtförmigen Aufbau handeln. Bevorzugt weist der Aufbau eine Abfolge von Schichten auf, welche aus der Gruppe bestehend aus Polyethylen-Aluminium-Polyethylenterephthalat, Polyvinylchlorid-Aluminium-Polyamid, Polypropylen-Aluminium-Lacke, Polyethylenterephthalat-Aluminium-Polypropylen und Lacke-Aluminium-Polyethylenterephthalat ausgewählt ist.

Erfindungsgemäß kann es bevorzugt sein, wenn die Deckschicht folienartig bzw. als Folie (Deckfolie) ausgebildet ist.

In einer besonders bevorzugten Ausführungsform weist die Blisterverpackung eine gasdurchlässige und keimdichte Abdeckschicht und eine gasdichte und insbesondere feuchtigkeitsdichte Deckschicht auf, wobei vorzugsweise die Abdeckschicht von der Deckschicht bedeckt bzw. versiegelt wird.

Bevorzugt weist die Verpackung eine Wandung auf, die die Befüllungskomponente, insbesondere einen Kunststoffkörper der Befüllungskomponente, formschlüssig und insbesondere abdichtend umgibt.

Die Komponente zur Befüllung der Verpackung ist in einer vorteilhaften Ausführungsform abklemmbar ausgebildet.

Bevorzugt ist die Komponente zur Befüllung der Verpackung aus einem mittels einer Injektionskanüle durchstechbaren Material wie beispielsweise Gummi hergestellt. Bei der Befüllungskomponente kann es sich daher zum Beispiel um einen Gummistopfen handeln. Alternativ kann die Befüllungskomponente auch als Septum ausgebildet sein.

In einer weiteren Ausführungsform ist die Komponente zur Befüllung der Verpackung mittels eines mit einer Injektionskanüle durchstechbaren Materials, vorzugsweise mittels einer Membran oder eines Septums, verschlossen.

Das medizinische Produkt ist unter Vakuum oder unter einem Unterdruck in der Verpackung bevorratet. Dies hat den Vorteil, dass bei der Befüllung der Verpackung mit einer Flüssigkeit ein Teil der Flüssigkeit aufgrund des in der Verpackung herrschenden Vakuums oder Unterdrucks ohne weiteres Zutun über die Befüllungskomponente in das Innere der Verpackung hineingezogen wird, bis ein Druckausgleich hergestellt ist. Dadurch ist eine vollständige Durchtränkung, insbesondere eine effiziente und vorzugsweise homogene zelluläre Kolonisierung, des medizinischen Produktes erzielbar. Dies ist insbesondere im Hinblick auf die begrenzte Verfügbarkeit von autologen Körperzellen zur Beladung des Produktes ein entscheidender Vorteil.

Wird beispielsweise die Verpackung mit einem flüssigen Spritzeninhalt befüllt, so wird ein Teil des Spritzeninhalts nach Einstechen der Spritzenkanüle in die Befüllungskomponente, welche bei dieser Ausführungsform zweckmäßigerweise aus einem mittels einer Injektionskanüle durchstechbaren Material gebildet ist oder mittels eines solchen Materials verschlossen ist, von selbst aus der Spritze in das Innere der Verpackung gezogen.

Das medizinische Produkt liegt aufgrund des in der Verpackung vorherrschenden Vakuums oder Unterdrucks komprimiert vor. Eine Relaxation bzw. Expansion des medizinischen Produktes beim Befüllen der Verpackung mit einer Flüssigkeit kann mit besonderem Vorteil zusätzlich die Saugwirkung des Produktes erhöhen und beispielsweise eine vollständige und insbesondere homogene Additivierung des Produktes mit Zellen und/oder Wirkstoffen begünstigen.

Weiterhin ist es erfindungsgemäß bevorzugt, wenn das medizinische Produkt in der Verpackung sterilisiert, vorzugsweise mittels einer γ-Bestrahlung sterilisiert, vorliegt. Dadurch ist eine Inkubation des Produktes mit einer Flüssigkeit, insbesondere einer zell- und/oder wirkstoffhaltigen Flüssigkeit, unter sterilen bzw. keimfreien Bedingungen möglich.

Die Flüssigkeit enthält vorzugsweise Wirkstoffe und/oder Zellen. Besonders bevorzugt handelt es sich bei der Flüssigkeit um eine zellhaltige Flüssigkeit, insbesondere zellhaltige Suspension.

Die Wirkstoffe können aus der Gruppe bestehend aus antimikrobielle, insbesondere antibiotische, Wirkstoffe, desinfizierende Wirkstoffe, entzündungshemmende Wirkstoffe, schmerzlindernde Wirkstoffe, geruchsbekämpfende Wirkstoffe, antiproliferative bzw. die Zellteilung hemmende Wirkstoffe, das Zellwachstum fördernde Wirkstoffe, Zellen rekrutierende Wirkstoffe, Zellen differenzierende Wirkstoffe, Gewebewachstum fördernde Wirkstoffe, chondroinduktive, d.h. das Knorpelgewebewachstum bzw. die Knorpelgeweberegeneration stimulierende, Wirkstoffe und Mischungen davon ausgewählt sein.

Bei den Zellen handelt es sich vorzugsweise um autologe Zellen, insbesondere um autologe Körperzellen. Autologe Zellen haben den Vorteil, dass schwerwiegende immunologische Reaktionen, insbesondere Abstoßungsreaktion, vermieden werden können.

Erfindungsgemäß können die Zellen jedoch auch allogenen Ursprungs sein.

Bevorzugt enthält die Flüssigkeit Zellen, welche aus der Gruppe bestehend aus Chondrozyten, Chondroblasten, Osteoblasten, Synovialzellen, Fibroblasten, Zellen des Periostgewebes, Zellen des Perichondriums, Stromazellen, insbesondere mesenchymale Stromazellen, Vorläuferzellen davon, Stammzellen davon, Stammzellen, insbesondere mesenchymale Stammzellen, und Kombinationen davon ausgewählt sind.

Des Weiteren ist das medizinische Produkt reversibel komprimierbar ausgebildet. Unter einem reversibel komprimierbaren Produkt soll im Sinne der vorliegenden Erfindung ein Produkt verstanden werden, welches unter Belastung, insbesondere Druck oder Unterdruck bzw. Vakuum, in einen komprimierten Zustand überführt werden kann, und nach Wegfall der Belastung wieder seine ursprüngliche Form und Größe annimmt bzw. im Wesentlichen wieder annimmt.

Das Produkt ist zweckmäßigerweise biokompatibel, insbesondere gegenüber Zellen, und vorzugsweise resorbierbar, insbesondere bioresorbierbar, d.h. in vivo resorbierbar, ausgebildet.

Das medizinische Produkt ist bevorzugt als Saugkörper ausgebildet oder weist bevorzugt einen Saugkörper auf. Der Saugkörper kann schwamm- oder schaumartig ausgebildet sein.

In einer weitergehenden Ausführungsform weist das medizinische Produkt eine schaumartige Komponente, membranartige Komponente und/oder faserförmige Komponente auf. Vorzugsweise weist das medizinische Produkt eine schaumartige Komponente und eine membranartige Komponente und/oder eine faserförmige Komponente auf.

Die schaumartige Komponente ist vorzugsweise aus einem Polymer, insbesondere einem synthetischen Polymer, insbesondere einem Kunststoff, gebildet. Eine Polymermischung ist erfindungsgemäß ebenfalls möglich.

Bevorzugt ist die schaumartige Komponente ein Polymerschaum, insbesondere ein synthetischer Polymerschaum, bevorzugt ein Kunststoffschaum.

In einer bevorzugten Ausführungsform handelt es sich bei der schaumartigen Komponente um ein geschäumtes Polymer oder gegebenenfalls eine geschäumte Polymermischung. Unter einem geschäumten Polymer bzw. einer geschäumten Polymermischung soll im Sinne der vorliegenden Erfindung ein Polymer bzw. eine Polymermischung verstanden werden, welches bzw. welche mittels dem Fachmann an sich bekannten Schäumungsverfahren wie beispielsweise des Reaktionsschaumguss-Verfahrens oder nach Art eines Reaktionsschaumguss-Verfahrens als Schaum oder schaumartige Matrix bzw. Struktur hergestellt werden kann.

Gemäß einer besonders bevorzugten Ausführungsform handelt es sich bei dem Polymer um Polyurethan. Das Polymer kann insbesondere ein lineares oder verzweigtes Polyurethan sein. Zusätzlich oder alternativ kann das Polymer ein aliphatisches oder aromatisches Polyurethan sein. Ein lineares und insbesondere aliphatisches Polyurethan ist erfindungsgemäß bevorzugt.

Das Polymer ist in einer weiteren Ausführungsform ein Polyurethan, welches ausgehend von wenigstens einer Polyolkomponente und wenigstens einer Polyisocyanatkomponente, bevorzugt mittels eines Schäumungsverfahren, insbesondere mittels eines Reaktionsschaumguss-Verfahrens bzw. nach Art eines Reaktionsschaumguss-Verfahrens, hergestellt bzw. herstellbar ist.

Unter einer Polyolkomponente soll im Sinne der vorliegenden Erfindung eine Alkoholverbindung mit zwei Hydroxygruppen (Diolkomponente) oder mehr Hydroxygruppen verstanden werden. In entsprechender Weise soll unter einer Polyisocyanatkomponente im Sinne der vorliegenden Erfindung eine Isocyanatverbindung mit zwei Isocyanatgruppen (Diisocyanatkomponente) oder mehr Isocyanatgruppen verstanden werden.

In einer besonders bevorzugten Ausführungsform ist das Polymer ein Polyurethan, welches ausgehend von einer Polyolkomponente, welche ausgewählt ist aus der Gruppe bestehend aus Ethylenglykol, Diethylenglykol, Propylenglykol, Trimethylolpropan, Glycerin, Neopentylglykol, 1,3-Propandiol, 1,4-Butandiol, 1,6-Hexandiol, Polycaprolactondiol, Polycaprolactontriol, Polyethylenglykol und Mischungen davon, und einer Polyisocyanatkomponente, welche ausgewählt ist aus der Gruppe bestehend aus Diphenylmethandiisocyanat, Toluol-2,4-diisocyanat, Naphthylendiisocyanat, Hexamethylendiisocyanat, Isophorondiisocyanat, 4,4'-Diisocyanatodicyclohexylmethan, Butandiisocyanat, Lysindiisocyanat und Mischungen davon, durch nukleophile Polyaddition hergestellt bzw. herstellbar ist.

Die schaumartige Komponente weist zweckmäßigerweise eine poröse, insbesondere offenporöse, Struktur auf. Bevorzugt besitzt die schaumartige Komponente eine Porengröße zwischen 10 und 450 µm, insbesondere 30 und 350 µm, vorzugsweise 50 und 250 µm. Diese Porengrößen haben sich als besonders vorteilhaft im Hinblick auf das Einwachsen bzw. Einsprossen von Zellen, Gewebe und Versorgungsgefäßen erwiesen.

In einer weiteren Ausführungsform besitzt die schaumartige Komponente Bereiche mit unterschiedlicher Porenstruktur, insbesondere unterschiedlichen Porengrößen und/oder -geometrien. Insbesondere kann die schaumartige Komponente einen Porengradienten besitzen.

In einer weiteren Ausführungsform weist die schaumartige Komponente, eine oberschenkelseitige Oberfläche, eine schienbeinseitige Oberfläche und eine gelenkkapselseitige Oberfläche auf. Unter einer oberschenkelseitigen Oberfläche soll im Sinne der vorliegenden Erfindung eine Oberfläche der schaumartigen Komponente verstanden werden, welche nach der Implantation des Produktes in ein Kniegelenk dem Oberschenkel (Femur) bzw. den Oberschenkel-Kondylen (femoralen Kondylen) zugewandt ist. Eine schienbeinseitige Oberfläche soll im Sinne der vorliegenden Erfindung eine Oberfläche der schaumartigen Komponente bedeuten, welche nach der Implantation des Produktes in ein Kniegelenk dem Schienbein (Tibia) bzw. den Schienbein-Kondylen (tibialen Kondylen) zugewandt ist. Unter einer gelenkkapselseitigen Oberfläche soll im Sinne der vorliegenden Erfindung eine Oberfläche der schaumartigen Komponente verstanden werden, welche nach der Implantation des Produktes der Gelenkkapsel (Randleiste) des Kniegelenks zugewandt ist.

Erfindungsgemäß kann es weiterhin vorgesehen sein, dass die schaumartige Komponente in Richtung der oberschenkelseitigen Oberfläche und/oder in Richtung der schienbeinseitigen Oberfläche abnehmende Porengrößen aufweist. Insbesondere können die oberschenkelseitige Oberfläche und/oder die schienbeinseitige Oberfläche derart kleine Porengrößen aufweisen, dass eine zelluläre Einsprossung nicht mehr möglich ist. In der Regel handelt es sich hierbei um Porengrößen < 1 µm. Derart kleine Porengrößen verleihen der oberschenkelseitigen Oberfläche und/oder der schienbeinseitigen Oberfläche gleitfähigere Eigenschaften, wodurch eine reibungslosere Artikulation mit dem Oberschenkelknochen (Femur) und/oder dem Schienbeinknochen (Tibia) möglich ist. Darüber hinaus kann auf diese Weise ein Zellenverlust über die oberschenkelseitige und/oder schienbeinseitige Oberfläche verhindert werden.

In einer weitergehenden Ausführungsform ist lediglich die gelenkkapselseitige Oberfläche der schaumartigen Komponente offenporös ausgebildet. Auf diese Weise ist eine kanalisierte Zelleinsprossung von der Gelenkkapsel eines Kniegelenks in die schaumartige Komponente möglich. Bei der Gelenkkapsel handelt es sich in der Regel um eine zellreiche Region, die an den äußeren Bereich eines Meniskus angrenzt.

In einer weiteren Ausführungsform weist die schaumartige Komponente einen Anteil zwischen 70 und 98 Gew.-%, insbesondere 80 und 97 Gew.-%, vorzugsweise 85 und 95 Gew.-%, auf, bezogen auf das Gesamtgewicht des Produktes.

Die schaumartige Komponente weist in einer besonders vorteilhaften Ausführungsform Kanäle bzw. kanalartige Strukturen, bevorzugt mit einer porösen Innenwandung, auf. Die Kanäle bzw. kanalartigen Strukturen können einen Innendurchmesser (lichte Weite) zwischen 100 und 600 µm, insbesondere 200 und 500 µm, bevorzugt von ca. 500 µm, aufweisen. Die Kanäle bzw. kanalartigen Strukturen stellen mit besonderem Vorteil Leit- oder Führungsschienen für einsprossende Zellen, einsprossendes Gewebe und/oder einsprossende Versorgungsgefäße, insbesondere Blutgefäße, dar.

Die membranartige Komponente besitzt vorzugsweise eine reiß- bzw. zugfeste Struktur und erlaubt insbesondere eine sichere und verschiebefeste bzw. dislokationsfeste Verankerung des medizinischen Produktes im Körper eines Patienten. Die membranartige Komponente ist insbesondere in der Lage, auf die schaumartige Komponente einwirkende Kräfte, wie sie typischerweise im Körper eines Patienten auftreten können, beispielsweise Scherkräfte, wenigstens teilweise abzufedern, um dadurch einer mechanischen Beschädigung der schaumartigen Komponente vorzubeugen. Des Weiteren besitzt die membranartige Komponente vorzugsweise ein Leit- bzw. Führungsschienenfunktion gegenüber Zellen wie beispielsweise Chondrozyten, Bindegewebe wie beispielsweise peripherem Bindegewebe und/oder natürlichen Versorgungsgefäßen wie beispielsweise Blutgefäßen.
Die membranartige Komponente kann insbesondere nicht porös oder im Wesentlichen nicht porös ausgebildet sein. Denkbar ist weiterhin, dass die membranartige Komponente derart kleine Porengrößen besitzt, beispielsweise < 1 µm, dass eine zelluläre Durchdringung bzw. Infiltration der membranartigen Komponente nicht möglich ist und die membranartige Komponente daher als undurchdringbare Barriere gegenüber Zellen wi rkt.

In einer weiteren Ausführungsform weist die membranartige Komponente einen Anteil zwischen 1,5 und 20 Gew.-%, insbesondere 2 und 15 Gew.-%, vorzugsweise 2 und 10 Gew.-%, auf, bezogen auf das Gesamtgewicht des Produktes.

In einer besonders bevorzugten Ausführungsform ist die membranartige Komponente teilweise, insbesondere nur teilweise, in der schaumartigen Komponente enthalten bzw. liegt teilweise, insbesondere nur teilweise, eingebettet in der schaumartigen Komponente vor. Insbesondere kann die membranartige Komponente teilweise, insbesondere nur teilweise, in die Struktur der schaumartigen Komponente integriert sein.

In einer besonders bevorzugten Ausführungsform ragt die membranartige Komponente in die schaumartige Komponente bzw. deren Struktur hinein. Mit anderen Worten ist es besonders bevorzugt, wenn die membranartige Komponente aus der schaumartigen Komponente bzw. deren Struktur herausragt bzw. wenn die membranartige Komponente teilweise nicht von der schaumartigen Komponente umgeben oder umhüllt ist.

Weiterhin ist es bevorzugt, wenn sich die membranartige Komponente, vorzugsweise durchgehend, in Längsrichtung der schaumartigen Komponente erstreckt.

Bevorzugt ragt die membranartige Komponente, vorzugsweise in Längsrichtung der schaumartigen Komponente, entlang einer gelenkkapselseitigen Oberfläche der schaumartigen Komponente heraus.

In einer weiteren Ausführungsform besitzt die membranartige Komponente Durchbrechungen, insbesondere in Form von Öffnungen, Löchern, Perforationen oder dergleichen. Entlang der Durchbrechungen ist die membranartige Komponente vorzugsweise von der schaumartigen Komponente durchsetzt bzw. durchdrungen. Dadurch ist eine feste Integration der membranartigen Komponente in der schaumartigen Komponente erzielbar.

Die membranartige Komponente ist in einer weiteren Ausführungsform biologischen, insbesondere xenogenen, bevorzugt tierischen, Ursprungs. Die membranartige Komponente kann beispielsweise porcinen, bovinen und/oder equinen Ursprungs sein. Bevorzugt ist die membranartige Komponente bovinen Ursprungs.

Die membranartige Komponente weist in einer weiteren Ausführungsform eine faserförmige Struktur oder eine Faserstruktur auf.

Vorzugsweise weist die membranartige Komponente ein Protein, insbesondere ein fibrilläres oder faserförmiges Protein, bevorzugt ein extrazelluläres Protein, auf.

Das Protein ist bevorzugt als Hauptbestandteil in der membranartigen Komponente enthalten. So kann die membranartige Komponente einen Proteinanteil von mehr als 50 Gew.-%, insbesondere von mehr als 75 Gew.-%, bevorzugt von mehr als 90 Gew.-%, aufweisen, bezogen auf das Gesamtgewicht der membranartigen Komponente.

Besonders bevorzugt ist die membranartige Komponente aus dem Protein gebildet.

Das Protein kann insbesondere ausgewählt sein aus der Gruppe bestehend aus Kollagen, Elastin, Retikulin, Fibronektin, Gelatine, Salze davon, Derivate davon und Mischungen davon.

In einer bevorzugten Ausführungsform ist das Protein Kollagen, insbesondere faserförmiges bzw. fibrilläres Kollagen, vorzugsweise ausgewählt aus der Gruppe bestehend aus Kollagen vom Typ I, Kollagen vom Typ II, Kollagen vom Typ III, Kollagen vom Typ V, Kollagen vom Typ XI, Salze davon, Derivate davon und Mischungen davon. Kollagen vom Typ I ist erfindungsgemäß besonders bevorzugt.

Bevorzugt handelt es sich bei der membranartigen Komponente um eine Membran, insbesondere um eine synthetische oder biologische Membran. Besonders bevorzugt handelt es sich bei der membranartigen Komponente um eine Proteinmembran, insbesondere Kollagenmembran. Eine besonders bevorzugte membranartige Komponente ist die von der Anmelderin kommerziell unter der Bezeichnung Lyoplant^{®} vertriebene Kollagenmembran. Bezüglich weiterer Merkmale und Vorteile zu dem Protein, insbesondere Kollagen, wird auf die bisherigen Ausführungen vollständig Bezug genommen.

In einer weiteren Ausführungsform handelt es sich bei der membranartigen Komponente um ein biologisches Gewebe, insbesondere um eine Gewebsschicht wie beispielsweise die Submucosa. Unter einem biologischen Gewebe soll im Sinne der vorliegenden Erfindung ein Gewebe verstanden werden, welches insbesondere in tierischen Organismen vorkommt oder mittels Geweberekonstruktionstechniken (tissue engineering) herstellbar ist.

In einer weiteren Ausführungsform handelt es sich bei der membranartigen Komponente um eine extrazelluläre Matrix (ECM), insbesondere ausgewählt aus der Gruppe bestehend aus Perikard, Peritoneum, Dünndarm-Submucosa, Magen-Submucosa, Harnblasen-Submucosa, Uterus-Submucosa, Serosa und Kombinationen davon.

In einer besonders bevorzugten Ausführungsform ist die membranartige Komponente ausgewählt aus der Gruppe bestehend aus Herzbeutel bzw. Perikard, Pericardium fibrosum, Pericardium serosum, Epikard, Plattenepithel, Tunica serosa, Muskel wie beispielsweise Myokard und Kombinationen davon.

In einer besonders bevorzugten Ausführungsform handelt es sich bei der membranartigen Komponente um Perikard, insbesondere tierisches Perikard. Das Perikard kann beispielsweise porcinen, bovinen und/oder equinen Urspungs sein. Vorzugsweise handelt es sich bei der membranartigen Komponente um bovines Perikard bzw. Rinderperikard.

Die faserförmige Komponente wirkt vorzugsweise ebenfalls als Leit- bzw. Führungsschiene für in die schaumartige Komponente einwachsende bzw. einsprossende Zellen.

Die faserförmige Komponente ist vorzugsweise in der schaumartigen Komponente, insbesondere ausschließlich in der schaumartigen Komponente, enthalten.

Bevorzugt weist die faserförmige Komponente das gleiche Material auf wie die membranartige Komponente bzw. ist aus dem gleichen Material gebildet wie die membranartige Komponente.

In einer vorteilhaften Ausführungsform handelt es sich bei der faserförmigen Komponente um hydrophobe Fasern. Hydrophobe Fasern haben den Vorteil, dass sich Zellen besser daran anhaften können, wodurch sich im Ergebnis die Leitschienenfunktion gegenüber Zellen verbessern lässt.

Bei der faserförmigen Komponente handelt es sich in einer weiteren Ausführungsform um biopolymerhaltige, insbesondere polysaccharidhaltige wie beispielsweise mucopolysaccharidhaltige und/oder proteinhaltige, vorzugsweise kollagenhaltige, Fasern.

Bevorzugt handelt es sich bei der faserförmigen Komponente um Fasern mit einem Proteinanteil, vorzugsweise Kollagenanteil, insbesondere einem Anteil an Kollagen vom Typ I, von mehr als 50 Gew.-%, insbesondere von mehr als 60 Gew.-%, bevorzugt von mehr als 70 Gew.-%, besonders bevorzugt von ca. 75 Gew.-%, bezogen auf das Einzelgewicht der Fasern.

In einer weiteren Ausführungsform handelt es sich bei der faserförmigen Komponente um Fasern biologischen, insbesondere xenogenen, vorzugsweise tierischen, Ursprungs. Beispielsweise kann es sich bei der faserförmigen Komponente um Fasern porcinen, bovinen und/oder equinen Ursprungs handeln. Bovine Fasern sind besonders bevorzugt.

In einer bevorzugten Ausführungsform handelt es sich bei der faserförmigen Komponente um Lyoplant^{®}-Fasern.

In einer weiteren bevorzugten Ausführungsform handelt es sich bei der faserförmigen Komponente um Perikardfasern, Epikardfasern, Myokardfasern und Kombinationen davon.

Die faserförmige Komponente weist bevorzugt einen Anteil zwischen 1 und 10 Gew.-%, insbesondere 1,5 und 7,5 Gew.-%, vorzugsweise 2 und 6 Gew.-%, auf, bezogen auf das Gesamtgewicht des Produktes.
In einer weiteren Ausführungsform besteht das Produkt aus einer schaumartigen Komponente, membranartigen Komponente und einer faserförmigen Komponente. Bezüglich weiterer Merkmale und Vorteile wird vollständig auf die bisherige Beschreibung Bezug genommen.

Das Produkt ist in einer weiteren vorteilhaften Ausführungsform mit Zellen, insbesondere autologen Zellen, bevorzugt autologen Körperzellen, beladen. Bezüglich geeigneter Zellen wird vollständig auf die bisherige Beschreibung Bezug genommen.

Des Weiteren kann das Produkt mit Wirkstoffen beladen sein. Bezüglich geeigneter Wirkstoffe wird ebenfalls vollständig auf die bisherige Beschreibung Bezug genommen.

Das Produkt, insbesondere die schaumartige Komponente, kann - abhängig von der jeweils zu behandelnden Indikation - in unterschiedlichen Formen und Größen vorliegen. Beispielsweise kann das medizinische Produkt scheibenförmig, insbesondere als kreisförmige Scheibe, zylindrisch, insbesondere als Flachzylinder, keil- oder streifenförmig ausgebildet sein.

Insbesondere kann das Produkt einen gekrümmten oder kurvenförmigen Verlauf aufweisen.

Bevorzugt ist das medizinische Produkt, insbesondere die schaumartige Komponente, halbkreisförmig, C-förmig, sichelförmig oder halbmondförmig ausgebildet. Insbesondere kann das Produkt als halbkreisförmiger, C-förmiger, sichelförmiger oder halbmondförmiger Keil oder als halbkreisförmige, C-förmige, sichelförmige oder halbmondförmige Scheibe ausgebildet sein.

Insbesondere kann das medizinische Produkt ein Kombinations- bzw. Kompositimplantat sein.

Bei dem medizinischen Produkt handelt es sich um ein Knorpelersatz- oder Knorpelteilersatzimplantat, insbesondere Meniskusersatz- oder Meniskusteilersatzimplantat, bevorzugt für den Innen- und/oder Außenmeniskus eines Kniegelenks.

Weitere Merkmale und Vorteile der Erfindung ergeben sich aus der nachfolgenden Beschreibung bevorzugter Ausführungsformen in Kombination mit den Figuren, den Figurenbeschreibungen, dem Beispiel sowie den Unteransprüchen. In diesen Ausführungsformen können einzelne Merkmale der Erfindung alleine oder in Kombination mit anderen Merkmalen verwirklicht sein. Die beschriebenen bevorzugten Ausführungsformen sind lediglich als beschreibende, keineswegs als in irgendeiner Weise limitierende Offenbarung zu verstehen. Sämtliche Figuren werden hiermit durch ausdrückliche Bezugnahme zum Inhalt dieser Beschreibung gemacht.

In den Figuren zeigen schematisch:
- Fig. 1: eine Ausführungsform der erfindungsgemäßen Verpackung
- Fig. 2: eine weitere Ausführungsform der erfindungsgemäßen Verpackung,
- Fig. 3: eine vergrößerte Darstellung des in der Figur 2 in der Verpackung enthaltenen medizinischen Produktes und
- Fig. 4: eine Seitenansicht des in den Figuren 2 und 3 dargestellten Produktes.

### 1. Herstellung eines erfindungsgemäßen Produktes zum Ersatz oder Teilersatz eines Meniskus

### 1.1 Vorbereitung der Rinderperikard-Membran

Die Membran wurde mit einer Kanüle (21 G x 2) perforiert. Anschließend wurde die Membran im Vakuum (2-10 mbar) für wenigstens sechs Stunden getrocknet. Danach wurde die Membran in eine Mischung aus Polycaprolactondiol (Mₙ=2000) und Isophorondiisocyanat im molaren Verhältnis 9:10 bei 50 °C eingelegt. Nach 90 Minuten wurden die Membran und die Mischung in ein Polyethylen-Wägeschiffchen überführt, in welchem die Membran mittels eines PTFE-ummantelten Rührfischchens fixiert wurde. Dort verblieb die Membran für insgesamt zwei Stunden, ehe die Membran in eine zweiteilige Silikonform (Formwerkzeug) eingelegt wurde.

### 1.2 Einlegen der Perikardmembran in ein Formwerkzeug

Eine zweiteilige Silikonform mit einer halbkreisförmigen Kavität wurde vor dem Einlegen der Perikardmembran für mindestens eine Stunde auf 50 °C temperiert. Anschließend wurde die Membran in die Silikonform eingelegt und mit Hilfe von drei Kanülen (21 G x 2) fixiert. Danach wurde die Silikonform von außen mit einem Klebeband verklebt.

### 1.3 Herstellung einer Polyolmischung

Die Polyole Polycaprolactondiol, Polycaprolactontriol, Polyethylenglykol und Butandiol wurden zur Verflüssigung auf eine Temperatur von ca. 40 °C temperiert, um sie zu verflüssigen. Danach wurden die Polyole in einem geeigneten, ebenfalls auf 40 °C temperierten Gefäß eingewogen und vermischt. Anschließend wurden die Additive DABCO, DC3042 und Lecithin hinzugegeben. Danach wurde eine homogene Mischung hergestellt. Schließlich wurden zu der Mischung kollagenhaltige Fasern aus der Muskelschicht des Herzens (Myokard) zu der Mischung hinzugegeben. Die erhaltene Mischung wurde erneut homogenisiert.

### 1.4 Zugabe der weiteren Komponenten

Zu der gemäß 1.3. hergestellten Mischung wurde Isophorondiisocyanat und nach kurzem Homogenisieren Methylal als Treibmittel hinzugegeben. Nach der Homogenisierung der Mischung erfolgte die Zugabe von Diazabicycloundecen.

Als die Mischung zu schäumen begann, wurde sie mittels einer 50 ml Spritze in die Silikonform injiziert. Hierzu wies die Silikonform geeignete Injektionsöffnungen auf.

Die Silikonform wurde für wenigstens fünf Stunden bei ca. 50 °C im Wärmeschrank gelagert. Danach ließ man die geschlossene Form für wenigstens zwei Stunden abkühlen.

Anschließend wurde die Siliconform geöffnet und das fertige Produkt vorsichtig entformt. Überschüssiges Polyurethan (Angüsse, Material in den Entlüftungskanälen, poröser Film im Innern des Halbkreissegmentes des Produktes) wurde entfernt. Das Produkt wurde in voll entsalztem Wasser für ca. zwei Minuten gewalkt und danach im Vakuum bis zur Gewichtskonstanz getrocknet.

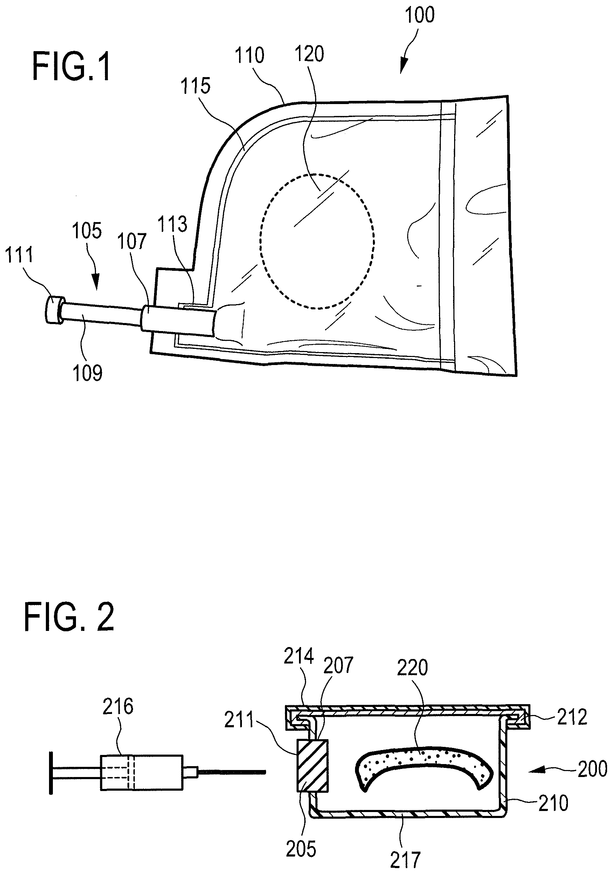

Das erhaltene Produkt besaß eine Form, wie sie schematisch in den Figuren 2 bis 4 dargestellt ist.

Fig. 1 zeigt schematisch eine als Kunststoffbeutel ausgebildete Verpackung 100, welche ein medizinisches Produkt 120 enthält.

Der Kunststoffbeutel 100 ist aus einer Kunststofffolie 110, insbesondere aus zwei Lagen davon, hergestellt. Bei der Kunststofffolie 110 kann es sich beispielsweise um eine Polypropylenfolie handeln.

Zur Befüllung mit einer Flüssigkeit, vorzugsweise mit einer autologe Chondrozyten enthaltenden Flüssigkeit, weist die Verpackung 100 eine als Port ausgeführte Befüllungskomponente 105 auf. Der Port 105 weist einen Kunststoffkörper 107 auf, der zumindest teilweise entlang eines Portschweißbereiches 113 in der Verpackung 100 eingeschweißt vorliegt. Der Port 105 ist abklemmbar ausgebildet und weist hierzu einen aus dem Kunststoffkörper 107 überstehenden abklemmbaren bzw. zusammendrückbaren Abschnitt 109 auf. Der Port 105 ist beispielsweise mittels eines Septums 111 überzogen. Zur Befüllung der Verpackung 100 mit einer Flüssigkeit kann beispielsweise die Kanüle einer Spritze in das Septum 111 gestochen und die Flüssigkeit durch Betätigung eines Spritzenkolbens über das Septum 111 in die Verpackung 100 ausgetragen werden.

Der Portschweißbereich 113 geht vorzugsweise fließend in einen randseitig verlaufenden Falzschweißbereich 115 über (Verschweißung entlang eines randseitig verlaufenden Falzes).

Das in Fig. 1 schematisch dargestellte medizinische Produkt 120 besitzt eine zylindrische, insbesondere flachzylindrische, Form. Indikationsabhängig kann das medizinische Produkt 120 selbstverständlich auch andere Formen und insbesondere Größen besitzen.

Das medizinische Produkt 120 ist vorzugsweise als Schaum, insbesondere Polymerschaum, ausgeführt. Besonders bevorzugt handelt es sich bei dem medizinischen Produkt 120 um einen Polyurethanschaum oder um geschäumtes Polyurethan.
Das medizinische Produkt 120 ist bevorzugt steril in der Verpackung 100 gelagert. Dadurch kann beispielsweise eine sterile zelluläre Besiedelung des Produktes 120 in der Verpackung 100 vorgenommen werden.

In der Verpackung 100 herrscht ein Vakuum oder Unterdruck, welches bzw. welcher das Produkt 120 in einem komprimierten Zustand hält. Das Produkt 120 ist reversibel komprimierbar ausgebildet, wodurch sich seine Saugwirkung beim Befüllen der Verpackung 100 mit einer Flüssigkeit vorzugsweise erhöht. Dadurch kann eine möglichst vollständige Durchtränkung des Produktes 120 mit der Flüssigkeit erreicht werden, was beispielsweise im Falle einer zellhaltigen Flüssigkeit eine möglichst rasche und insbesondere homogene zelluläre Beladung des Produktes 120 begünstigen kann.

Die Fig. 2 zeigt schematisch eine weitere Ausführungsform einer erfindungsgemäßen Verpackung 200. Die Verpackung 200 ist als Tiefziehblisterverpackung ausgebildet. Die Tiefziehblisterverpackung 200 umfasst ein Kunststofftiefziehformteil 210, beispielsweise aus Polyethylenterephthalat, mit einer Gehäuseform, in welchem ein medizinisches Produkt 220 gelagert ist. Das Kunststofftiefziehformteil 210 wird bevorzugt von einer gasdurchlässigen und insbesondere keimdichten Abdeckschicht 212 verschlossen. Die Abdeckschicht 212 kann beispielsweise aus dem kommerziell unter der Bezeichnung Tyvek^{®} erhältlichen Material gebildet sein. Die Abdeckschicht 212 wiederum wird bevorzugt von einer gasundurchlässigen und insbesondere flüssigkeitsundurchlässigen Deckschicht 214, vorzugsweise aus Aluminium oder einem Verbundmaterial mit Aluminium, bedeckt bzw. versiegelt. Sowohl die Abdeckschicht 212 als auch die Deckschicht 214 sind vorzugsweise folienartig ausgebildet.

Zur Befüllung mit einer Flüssigkeit, beispielsweise mit Hilfe einer Spritze 216, besitzt die Verpackung 200 beispielsweise eine mit einem Septum 211 versiegelte Befüllungskomponente 205 mit einem Kunststoffkörper 207, welcher vorzugsweise formschlüssig und insbesondere abdichtend in der Wandung 217 des Kunststofftiefziehformteils 210 integriert ist.

Das in Fig. 2 dargestellte medizinische Produkt 220 besitzt vorzugsweise die Form eines halbkreis- oder C-förmigen Keils und eignet sich in dieser Form insbesondere zur Behandlung von Meniskusschäden, insbesondere eines Kniegelenkes. Selbstverständlich sind, abhängig von der zu behandelnden medizinischen Indikation, auch andere Formen für das medizinische Produkt denkbar.

Auch das in der Verpackung 200 bevorratete Produkt 220 liegt bevorzugt sterilisiert vor und liegt darüber hinaus aufgrund eines in der Verpackung 200 vorherrschenden Unterdrucks bzw. Vakuums ebenfalls in einem komprimierten Zustand vor.

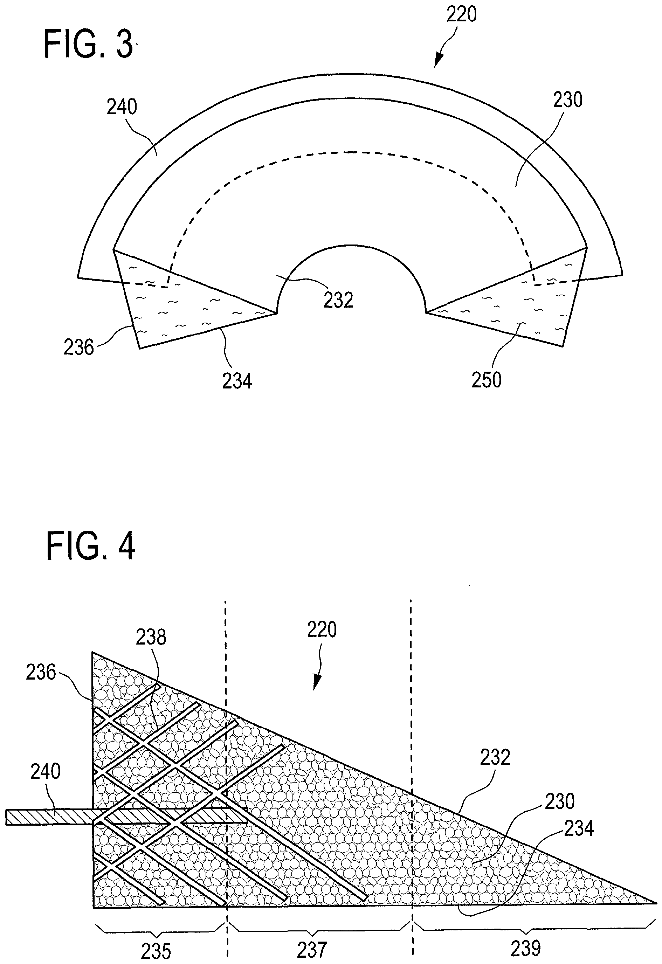

Fig. 3 zeigt schematisch das medizinische Produkt 220 aus der Figur 2 in vergrößerter Darstellung. Das medizinische Produkt 220 weist eine schaumartige Komponente 230, eine membranartige Komponente 240 und eine faserförmige Komponente 250 auf.

Die schaumartige Komponente 230 besitzt in der Regel eine offenporöse Struktur und dient vorzugsweise als Trägermatrix für einwachsende bzw. einsprossende Zellen wie beispielsweise Chondrozyten, Vorläuferzellen davon und/oder Stammzellen davon.

Bei der schaumartigen Komponente 230 handelt es sich bevorzugt um ein geschäumtes Polymer bzw. um einen Polymerschaum, insbesondere um geschäumtes Polyurethan bzw. einen Polyurethanschaum.

Die schaumartige Komponente 230 besitzt bevorzugt die Form eines halbkreisförmigen oder C-förmigen Keils oder einer halbkreisförmigen oder C-förmigen Scheibe. Darüber hinaus sind jedoch, abhängig von der jeweiligen medizinischen Indikation, auch andere Formen denkbar.

Das in der Figur 3 (und der Figur 2) schematisch dargestellte Produkt 220 eignet sich insbesondere als Meniskusersatz- oder Meniskusteilersatzimplantat, bevorzugt als Ersatz- oder Teilersatzimplantat für den Innen- und/oder Außenmeniskus eines Kniegelenks, vorzugsweise eines menschlichen Kniegelenks.

Bevorzugt ist die Oberfläche 232 nach Implantation des Produktes 220 in ein Kniegelenk den Oberschenkelkondylen (femorale Kondylen) zugewandt (oberschenkelseitige Oberfläche). Die Oberfläche 234 ist nach Implantation des Produktes 220 in ein Kniegelenk vorzugsweise den Schienbeinkondylen (tibiale Kondylen) zugewandt (schienbeinseitige Oberfläche). Die Oberfläche 236 ist nach Implantation des Produktes 220 vorzugsweise der Gelenkkapsel eines Kniegelenks zugewandt (gelenkkapselseitige Oberfläche).

Die membranartige Komponente 240 ragt entlang der Oberfläche 236 aus der schaumartigen Komponente 230.

Die membranartige Komponente 240 besitzt vorzugsweise im Wesentlichen den gleichen gekrümmten Verlauf wie die schaumartige Komponente 230.

Bevorzugt ist die membranartige Komponente 240 eine tierische Membran wie beispielsweise Rinderperikard.

Bei der faserförmigen Komponente 250 handelt es sich bevorzugt um proteinhaltige, insbesondere kollagenhaltige, Fasern wie beispielsweise Myokardfasern. Die faserförmige Komponente 250 ist vorzugsweise ausschließlich in der schaumartigen Komponente 230 enthalten.

Wie bereits in der bisherigen Beschreibung ausgeführt, können sowohl die membranartige Komponente 240 als auch die faserförmige Komponente 250 mit besonderem Vorteil als Leit- bzw. Führungsschiene für Zellen, Körpergewebe und/oder natürliche Versorgungsgefäße in Richtung der schaumartigen Komponente 230 wirken. Dadurch ist eine rasche, quantitative und insbesondere homogene zelluläre Besiedelung der schaumartigen Komponente 230, eine bindegewebsbasierte Verankerung des Produktes 220 in einer Körperdefektzone und/oder eine ausreichende Nährstoffversorgung möglich. Dies begünstigt, insbesondere beschleunigt, die Neuenstehung von Knorpelgewebe in einer zu versorgenden Knorpelzone.

Um eine zelluläre Einsprossung, Gewebeeinsprossung und/oder Gefäßeinsprossung in die schaumartige Komponente 230 zusätzlich zu verbessern, kann die schaumartige Komponente 230 Kanäle bzw. kanalartige Strukturen 238 aufweisen (Figur 4, faserförmige Komponente ist in der Figur 4 nicht dargestellt). Beispielsweise können die Kanäle 238 lediglich in einem äußeren Bereich 235 und einem mittleren Bereich 237 der schaumartigen Komponente 230 ausgebildet sein, während ein zentraler Bereich 239 der schaumartigen Komponente 230 vorzugsweise frei von derartigen Kanälen 238 ist. Dadurch besitzt der zentrale Bereich 239 eine im Verhältnis zum peripheren Bereich 235 und mittleren Bereich 237 kompaktere Struktur, was insbesondere bei einem als Meniskusersatz- oder -teilersatzimplantat ausgebildeten Produkt 220 von Vorteil ist, bei welchem der zentrale Bereich 239 besonders hohen Belastungskräften, insbesondere Scherkräften, ausgesetzt ist.

Die erfindungsgemäße Verpackung erlaubt in besonders vorteilhafter Weise die Bereitstellung eines mit Zellen und/oder Wirkstoffen additivierten medizinischen Produktes. Bevorzugt wird die erfindungsgemäße Verpackung dazu verwendet, ein mit Zellen, vorzugsweise autologen Körperzellen wie beispielsweise Chondrozyten, beladenes Produkt, insbesondere zur Regeneration und/oder Rekonstruktion von Knorpelschäden wie beispielsweise Meniskusschäden, zur Verfügung zu stellen. Hierzu wird das in der Verpackung bevorratete medizinische Produkt über die Befüllungskomponente mit einer zellhaltigen Flüssigkeit, in der Regel einer zellhaltigen Suspension, inkubiert. Dadurch kann eine ausreichende und insbesondere homogene zelluläre Besiedelung des Produktes bereits während seiner vorzugsweise sterilen Lagerung in der Verpackung erzielt werden. Zur Beschleunigung der zellulären Besiedelung kann es beispielsweise vorgesehen sein, die Verpackung über ihre Befüllungskomponente zusätzlich mit geeigneten Nährstofflösungen zu befüllen.

Im Ergebnis stellt die erfindungsgemäße Verpackung daher eine Befüllungsvorrichtung für das darin befindliche medizinische Produkt dar, welche einfach und insbesondere steril herstellbar ist und mit besonderem Vorteil eine für den Mediziner vorteilhafte und bequeme Möglichkeit bietet, das medizinische Produkt bereits während seiner Bevorratung in der Verpackung effizient und unter keimfreien Bedingungen mit einer in der Regel nur begrenzt verfügbaren Menge an autologen Körperzellen wie beispielsweise Chondrozyten zu besiedeln. Nachdem sich die Zellen in der Verpackung ausreichend vermehrt haben, kann das Produkt aus der Verpackung entnommen werden und unmittelbar in den Körper eines Patienten implantiert werden, ohne dass zusätzliche Maßnahmen erforderlich wären, die gegebenenfalls die Integrität des Produktes, die Menge an Zellen in dem Produkt und/oder die Keimfreiheit des Produktes beeinträchtigen könnten.

## Patentansprüche

1. Verpackung (100; 200), insbesondere nach Art eines Beutels, enthaltend ein reversibel komprimierbar ausgebildetes medizinisches Produkt (120; 220), wobei die Verpackung (100; 200) eine Komponente (105; 205), vorzugsweise einen Port, zur Befüllung der Verpackung (100; 200) mit einer Flüssigkeit, vorzugsweise zellhaltigen Flüssigkeit, aufweist, **dadurch gekennzeichnet, dass** das medizinische Produkt (120; 220) ein Knorpelersatz- oder Knorpelteilersatzimplantat ist und unter Vakuum oder unter einem Unterdruck in der Verpackung (100; 200) bevorratet ist und aufgrund des Vakuums oder Unterdrucks komprimiert vorliegt.

2. Verpackung (100; 200) nach Anspruch 1, **dadurch gekennzeichnet, dass** die Verpackung (100; 200) ein Kunststoffbeutel ist.

3. Verpackung (100; 200) nach Anspruch 1 oder 2, **dadurch gekennzeichnet, dass** die Verpackung (100; 200) aus einer Kunststofffolie, insbesondere einer zweilagigen Kunststofffolie, hergestellt ist.

4. Verpackung (100; 200) nach einem der vorhergehenden Ansprüche, insbesondere nach Anspruch 3, **dadurch gekennzeichnet, dass** die Verpackung (100; 200), insbesondere die Kunststofffolie, aus einem Kunststoff hergestellt ist, welcher ausgewählt ist aus der Gruppe bestehend aus Polyethylen, Polypropylen, Copolymere davon, insbesondere Ethylen-Vinylalkohol, und Mischungen, insbesondere Blends, davon.

5. Verpackung (100; 200) nach einem der vorhergehenden Ansprüche, **dadurch gekennzeichnet, dass** die Befüllungskomponente (105; 205) einen Kunststoffkörper (107; 207) umfasst, der zwischen zwei Lagen, insbesondere zwischen zwei Kunststofffolienlagen, der Verpackung (100; 200) eingeschweißt ist.

6. Verpackung (100; 200) nach einem der vorhergehenden Ansprüche, **dadurch gekennzeichnet, dass** die Verpackung (100; 200) verschweißt ist, insbesondere entlang eines randseitig verlaufenden Falzes.

7. Verpackung (100; 200) nach einem der vorhergehenden Ansprüche, **dadurch gekennzeichnet, dass** die Verpackung (100; 200) einen Portschweißbereich (113) und einen Falzschweißbereich (115) aufweist, wobei der Portschweißbereich (113) und der Falzschweißbereich (115) vorzugsweise fließend in einander übergehen.

8. Verpackung (100; 200) nach Anspruch 1, **dadurch gekennzeichnet, dass** die Verpackung (100; 200) als Blisterverpackung, insbesondere als Tiefziehblisterverpackung, ausgebildet ist.

9. Verpackung (100; 200) nach Anspruch 8, **dadurch gekennzeichnet, dass** die Blisterverpackung eine gasdurchlässige und insbesondere keimdichte Abdeckschicht (212) und eine gasdichte und insbesondere flüssigkeitsdichte Deckschicht (214) aufweist, wobei die Abdeckschicht (212) vorzugsweise von der Deckschicht (214) bedeckt bzw. versiegelt ist.

10. Verpackung (100; 200) nach einem der vorhergehenden Ansprüche, **dadurch gekennzeichnet, dass** die Komponente (105; 205) zur Befüllung der Verpackung (100; 200) mittels eines mit einer Injektionskanüle durchstechbaren Materials, vorzugsweise mittels einer Membran oder eines Septums, verschlossen ist.

11. Verpackung (100; 200) nach einem der vorhergehenden Ansprüche, **dadurch gekennzeichnet, dass** das medizinische Produkt (120; 220) ein Meniskusersatz- oder Meniskusteilersatzimplantat ist.

## Claims

1. A package (100; 200), in particular in the form of a bag, containing a reversibly compressible medical product (120; 220), said package (100; 200) having a component (105; 205), preferably a port, for filling the package (100; 200) with a liquid, preferably a cell-containing liquid, **characterized in that** the medical product (120; 220) is an implant for replacement of cartilage or partial replacement of cartilage, and is stored under vacuum or at a negative pressure in the package (100; 200), and is present in a compressed state on account of the vacuum or negative pressure.

2. The package (100; 200) according to claim 1, **characterized in that** the package (100; 200) is a plastic bag.

3. The package (100; 200) according to claim 1 or 2, **characterized in that** the package (100; 200) is produced from a plastic film, in particular a two-ply plastic film.

4. The package (100; 200) according to any one of the preceding claims, in particular according to claim 3, **characterized in that** the package (100; 200), in particular the plastic film, is produced from a plastic that is chosen from the group consisting of polyethylene, polypropylene, copolymers thereof, in particular ethylene vinyl alcohol, and mixtures, in particular blends, thereof.

5. The package (100; 200) according to any one of the preceding claims, **characterized in that** the filling component (105; 205) comprises a plastic body (107; 207) which is welded in between two plies, in particular between two plastic film plies, of the package (100; 200).

6. The package (100; 200) according to any one of the preceding claims, **characterized in that** the package (100; 200) is welded, in particular along a fold running at the edge.

7. The package (100; 200) according to any one of the preceding claims, **characterized in that** the package (100; 200) has a port weld area (113) and a fold weld area (115), wherein the port weld area (113) and the fold weld area (115) merge preferably seamlessly into each other.

8. The package (100; 200) according to claim 1, **characterized in that** the package (100; 200) is designed as a blister pack, in particular as a thermoformed blister pack.

9. The package (100; 200) according to claim 8, **characterized in that** the blister pack has a gas-permeable and, in particular, germ-proof cover layer (212), and has a gastight and, in particular, liquid-tight top layer (214), wherein the cover layer (212) is preferably covered and sealed by the top layer (214).

10. The package (100; 200) according to any one of the preceding claims, **characterized in that** the component (105; 205) for filling the package (100; 200) is closed by means of a material that can be pierced with an injection needle, preferably by means of a membrane or a septum.

11. The package (100; 200) according to any one of the preceding claims, **characterized in that** the medical product (120; 220) is an implant for replacement of the meniscus or partial replacement of the meniscus.

## Revendications

1. Emballage (100; 200), en particulier à la manière d'un sac, contenant un produit médicinal comprimable de façon réversible (120; 220), dans lequel l'emballage (100; 200) présente un composant (105; 205), de préférence un orifice, pour le remplissage de l'emballage (100; 200) avec un liquide, de préférence un liquide contenant des cellules, **caractérisé en ce que** le produit médicinal (120; 220) est un implant de remplacement de cartilage ou un implant de remplacement partiel de cartilage et est stocké dans l'emballage (100; 200) sous vide ou sous une dépression et se trouve comprimé en raison du vide ou de la dépression.

2. Emballage (100; 200) selon la revendication 1, **caractérisé en ce que** l'emballage (100; 200) est un sac en matière plastique.

3. Emballage (100; 200) selon la revendication 1 ou 2 **caractérisé en ce que** l'emballage (100; 200) est fabriqué à partir d'un film de matière plastique, en particulier d'un film de matière plastique à deux couches.

4. Emballage (100; 200) selon l'une quelconque des revendications précédentes, en particulier selon la revendication 3, **caractérisé en ce que** l'emballage (100; 200), en particulier le film en matière plastique, est fabriqué en une matière plastique, qui est sélectionnée dans le groupe composé du polyéthylène, du polypropylène, de copolymères de ceux-ci, en particulier de l'éthylène-alcool vinylique, et de mélanges, en particulier de mixtures, de ceux-ci.

5. Emballage (100; 200) selon l'une quelconque des revendications précédentes, **caractérisé en ce que** le composant de remplissage (105; 205) comprend un corps en matière plastique (107; 207), qui est soudé entre deux couches, en particulier entre deux couches de film en matière plastique, de l'emballage (100; 200).

6. Emballage (100; 200) selon l'une quelconque des revendications précédentes, **caractérisé en ce que** l'emballage (100; 200) est soudé, en particulier le long d'un pli s'étendant sur le bord.

7. Emballage (100; 200) selon l'une quelconque des revendications précédentes, **caractérisé en ce que** l'emballage (100; 200) présente une zone de soudage d'orifice (113) et une zone de soudage de pli (115), dans lequel la zone de soudage d'orifice (113) et la zone de soudage de pli (115) se prolongent l'une dans l'autre de préférence de façon continue.

8. Emballage (100; 200) selon la revendication 1, **caractérisé en ce que** l'emballage (100; 200) est réalisé sous la forme d'un emballage à blister, en particulier d'un emballage à blister embouti.

9. Emballage (100; 200) selon la revendication 8, **caractérisé en ce que** l'emballage à blister présente une couche de recouvrement (212) perméable au gaz et en particulier étanche aux germes et une couche de couverture (214) étanche au gaz et en particulier étanche au liquide, dans lequel la couche de recouvrement (212) est de préférence recouverte ou scellée par la couche de couverture (214).

10. Emballage (100; 200) selon l'une quelconque des revendications précédentes, **caractérisé en ce que** le composant (105; 205) pour le remplissage de l'emballage (100; 200) est fermé au moyen d'un matériau pouvant être transpercé par une canule d'injection, de préférence au moyen d'une membrane ou d'un septum.

11. Emballage (100; 200) selon l'une quelconque des revendications précédentes, **caractérisé en ce que** le produit médicinal (120; 220) est un implant de remplacement de ménisque ou un implant de remplacement partiel de ménisque.
